# EUROPEAN PATENT APPLICATION

(11) **EP 4 353 711 A1**
(43) Date of publication of application: **17.04.2024**
(21) Application number: 22382968.0
(22) Date of filing: 11.10.2022
(51) Int. Cl.: C07D 215/26, A61P 25/00, A61K 31/4709

(54) **QUINOLIN-2-YL NITRONES FOR THE TREATMENT AND PREVENTION OF STROKE**

(71) Applicant: Consejo Superior De Investigaciones Científicas, 28006 Madrid (ES); Universidad Camilo José Cela, 28692 Villanueva de la Cañada (ES)
(72) Inventor: MARCO CONTELLES, José Luis, Madrid (ES); MARTÍNEZ MURILLO, Ricardo Pedro, Madrid (ES); LÓPEZ MUÑOZ, Francisco, Madrid (ES)
(74) Representative: Pons

(57) **Abstract**

The invention relates to quinolin-2-yl nitrone derivatives of formula I, wherein R₁ to R₇ groups have the meanings defined in the description, and to their use for the treatment and/or prevention of a vascular disease, particularly for the treatment and/or prevention of cerebral ischemia or stroke.

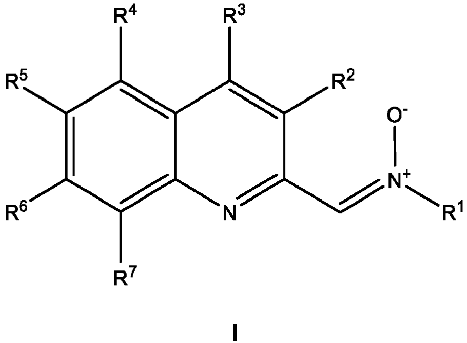

## Description

The invention relates to quinolin-2-yl nitrone derivatives of formula **I.** The invention also relates to their use for the treatment and/or prevention of a vascular disease, particularly for the treatment and/or prevention of cerebral ischemia or stroke.

### BACKGROUND ART

Oxidative stress is possibly one of the most important molecular events occurring during and after cerebral ischemic injury, and in particular their induction of damage to the membrane lipids (Alper, G. et al. Eur. Nueropsychopharmacol. 1999, 9, 247-252). Current research efforts directed to find an efficient therapies for stroke, overcoming the limitations of current clinically administered thrombolytic recombinant tissue plasminogen activator (rtPA) or thrombectomy-based therapeutic approaches, are mainly focused on the identification of new, more efficient neuroprotective agents able to block and scavenge reactive oxygenated species (ROS). This is due to the high susceptibility of neuronal membranes to be attacked by ROS through the allylic carbon of polyunsaturated fatty acids, main component of these brain structures. The resulting lipid hydroperoxide species are very unstable *in vivo* and, in the presence of bio-metals, such as iron salts, can be decomposed to new ROS, which will trigger further free radical cascade reactions. Potential consequences of membrane lipids damage include changes in fluidity, permeability, and orientation of proteins embedded in the bilayer of the plasma membrane, along with other cellular endo-membranes, leading finally to cell death in the brain tissue.

In this context, free radical scavengers, such as nitrones (Floyd, R. A.; Kopke, R. D. Choi, C. H.; Foster, S. B.; Doblas, S.; Towner, R. A. Nitrones as therapeutics. Free Radic. Biol. Med. 2008, 45, 1361-1374), have proved to be efficient neuroprotective agents in experimental ischemia studies. However, to date, no nitrone has been marketed for stroke treatment, due to failed clinical studies. This is the case of (Z)-α-phenyl-*N*-*tert*-butylnitrone (PBN) (Kalyanaraman, B.; Joseph, J.; Parthasarathy, S. The spin trap, α-phenyl N-tert-butylnitrone, inhibits the oxidative modification of low density lipoprotein (FEBS Lett. 1991, 280, 17-20), a simple but largely investigated nitrone that inhibits lipoprotein oxidation, reduces the oxidative damage to erythrocytes and the phenylhydrazine-induced peroxidation of lipids, and protects gerbils and mice from brain stroke and MPTP toxicity, respectively. In spite of this, nitrone PBN mechanism of action is still not clear. It does not seem due to its ability to act as a ROS trap, but to the suppression of inducible nitric oxide (NO) synthase expression, cytokine accumulation and apoptosis. On the other hand, the formation of NO from PBN spin adducts could also play a role in the observed effects in the central nervous system. Another well-known nitrone is sodium (*Z*)-4-((*tert-*butyloxidoazanylidene)methyl)benzene-1,3-disulfonate (NXY-059) (Kuroda, S.; Tsuchidate, R.; Smith, M. L.; Maples, K. R.; Siesjo, B. K. Neuroprotective effects of a novel nitrone, NXY-059, after transient focal cerebral ischemia in the rat. J. Cereb. Blood Flow Metab. 1999, 19, 778-787), which based on its good neuroprotection in suitable animal models of transient and permanent focal ischemia, it was assayed in several clinical studies, unfortunately without success. In spite of these unfulfilled expectations, the current efforts devoted to discover new nitrones for the treatment of stroke show and proves that the antioxidant and neuroprotective strategy is still a choice for the development of new drugs for stroke.

In this context, in the present invention new nitrones have been synthesized and biologically evaluated as potential drugs for cerebral ischemia treatment.

In previous studies (Z)-*N*-benzyl-1-(2-chloro-6-methylquinolin-3-yl)methanimine oxide RP19 (Chioua M, Sucunza D, Soriano E, Hadjipavlou-Litina D, Alcázar A, Ayuso I, Oset-Gasque MJ, Gonzalez MP, Monjas L, Rodríguez-Franco MI, Marco-Contelles J, Samadi A. α-aryl-N-alkyl nitrones, as potential agents for stroke treatment: synthesis, theoretical calculations, antioxidant, anti-inflammatory, neuroprotective, and brain-blood barrier permeability properties. J Med Chem. 2012, 55, 153-168) as potent antioxidant and neuroprotective agents. Remarkably, in the case of RP19, the biological analysis showed that this nitrone induced long-term cell viability after 5 d of recovery after oxygen-glucose deprivation in primary neuronal cultures, and significantly reduced ROS and lipid peroxidation production. Furthermore, pharmacological treatment with nitrone RP19 of reperfused animals after both global and focal ischemia, at the dose that was demonstrated to be neuroprotective in neuronal cultures, significantly decreased neuronal death and apoptosis induction, reduced the size of infarct, and improved the neurodeficit scores after 48 h or 5 days of reperfusion after ischemia.

Considering the above, it would be desirable to improve the promising biological results gathered from nitrone RP19 to obtain more potent, efficient antioxidant and neuroprotective compounds for the treatment and/or prevention of a vascular diseases such as cerebral ischaemia and stroke.

### SUMMARY OF THE INVENTION

A first aspect of the present invention related to a compound of formula **I**: 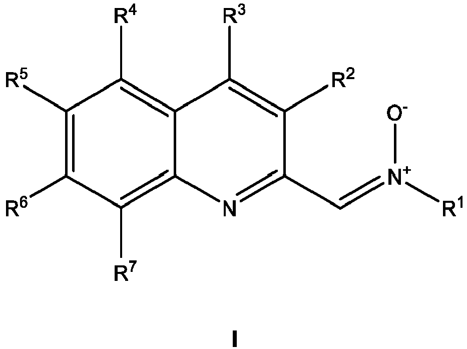 wherein:
R¹ independently represents C₁₋₄ alkyl, phenyl or benzyl (Bn);
each R² to R⁷ independently represents H, C₁₋₄ alkyl, phenyl, halogen, -OR⁸ or -NHR⁸; each R⁸ independently represents H or C₁₋₄ alkyl, wherein C₁₋₄ alkyl is optionally substituted by one or more R⁹.
each R⁹ independently represents halogen, -OH, -OC₁₋₄ alkyl, NH₂, -NH(C₁₋₄ alkyl) or Cy₁; and
Cy₁ represents a 5-to-8-member ring, saturated, partially unsaturated or aromatic, which optionally contains from 1 to 3 heteroatoms selected from N, O, Se and/or S; wherein Cy₁ may be attached to rest of the molecule through any available C or N atom, and wherein Cy₁ is optionally substituted by one or more R¹⁰;
each R¹⁰ independently represent -C₁₋₄ alkyl optionally substituted by one or more R¹¹; and
each R¹¹ independently represents -C₂₋₄ alkynyl.

In another embodiment the invention relates to the compound of formula I as defined above, wherein R¹ represents H, -C₁₋₄ alkyl or benzyl, and preferably R¹ represents methyl (-Me), *tert*-butyl (-*t*-Bu) or benzyl (-Bn).

In another embodiment the invention relates to the compound of formula **I** as defined above, wherein each R² to R⁶ independently represents H.

In another embodiment the invention relates to the compound of formula **I** as defined above, wherein each R² to R⁶ represents H.

In another embodiment the invention relates to the compound of formula **I** as defined above, wherein:
R¹ represents H, -C₁₋₄ alkyl or benzyl, and preferably R¹ represents methyl (-Me), *tert-*butyl (-*t*-Bu) or benzyl (-Bn); and
each R² to R⁶ independently represents H, and preferably each R² to R⁶ represents H.

In another embodiment the invention relates to the compound of formula **I** as defined above, wherein R⁷ represents H or -OR⁸.

In another embodiment the invention relates to the compound of formula **I** as defined above, wherein R⁸ represents C₁₋₄ alkyl optionally substituted by one or more R⁹.

In another embodiment the invention relates to the compound of formula **I** as defined above, wherein:
R⁷ represents -OR⁸; and
R⁸ represents C₁₋₄ alkyl optionally substituted by one or more R⁹.

In another embodiment the invention relates to the compound of formula **I** as defined above, wherein R⁹ represents Cy₁.

In another embodiment the invention relates to the compound of formula **I** as defined above, wherein Cy₁ represents a saturated 5- member ring, which contains 1 or 2 N heteroatoms; wherein Cy₁ may be attached to rest of the molecule through any available C or N atom, and wherein Cy₁ is optionally substituted by one or more R¹⁰.

In another embodiment the invention relates to the compound of formula **I** as defined above, wherein:
R⁹ represents Cy₁; and
Cy₁ represents a saturated 5- member ring, which contains 1 or 2 N heteroatoms; wherein Cy₁ may be attached to rest of the molecule through any available C or N atom, and wherein Cy₁ is optionally substituted by one or more R¹⁰.

In another embodiment the invention relates to the compound of formula **I** as defined above, wherein R¹⁰ represents -C₁₋₄ alkyl optionally substituted by one or more R¹¹, and preferably R¹⁰ represent methyl substituted by one R¹¹.

In another embodiment the invention relates to the compound of formula **I** as defined above, wherein:
R⁹ represents Cy₁;
Cy₁ represents a saturated 5- member ring, which contains 1 or 2 N heteroatoms; wherein Cy₁ may be attached to rest of the molecule through any available C or N atom, and wherein Cy₁ is optionally substituted by one or more R¹⁰; and
R¹⁰ represents -C₁₋₄ alkyl optionally substituted by one or more R¹¹, and preferably R¹⁰ represent methyl substituted by one R¹¹.

In another embodiment the invention relates to the compound of formula **I** as defined above, wherein:
R¹ represents H, -C₁₋₄ alkyl or benzyl, and preferably R¹ represents methyl (-Me), *tert-*butyl (-*t*-Bu) or benzyl (-Bn);
R² to R⁶ represents H; and
R⁷ represents -OR⁸.

In another embodiment the invention relates to the compound of formula **I** as defined above, wherein:
R¹ represents H, -C₁₋₄ alkyl or benzyl, and preferably R¹ represents methyl (-Me), *tert-*butyl (-*t*-Bu) or benzyl (-Bn);
R² to R⁶ represents H,
R⁷ represents -OR⁸; and
R⁸ represents C₁₋₄ alkyl, wherein C₁₋₄ alkyl is optionally substituted by one or more R⁹.

In another embodiment the invention relates to the compound of formula **I** as defined above, wherein:
R¹ represents H, -C₁₋₄ alkyl or benzyl, and preferably R¹ represents methyl (-Me), *tert-*butyl (-*t*-Bu) or benzyl (-Bn);
R² to R⁶ represents H,
R⁷ represents -OR⁸;
R⁸ represents C₁₋₄ alkyl, wherein C₁₋₄ alkyl is optionally substituted by one or more R⁹ and
R⁹ represents Cy₁.

In another embodiment the invention relates to the compound of formula **I** as defined above, wherein:
R¹ represents H, -C₁₋₄ alkyl or benzyl, and preferably R¹ represents methyl (-Me), *tert-*butyl (-*t*-Bu) or benzyl (-Bn);
R² to R⁶ represents H,
R⁷ represents -OR⁸;
R⁸ represents C₁₋₄ alkyl, wherein C₁₋₄ alkyl is optionally substituted by one or more R⁹; R⁹ represents Cy₁; and
Cy₁ represents a saturated 5- member ring, which contains 1 or 2 N heteroatoms; wherein Cy₁ may be attached to rest of the molecule through any available C or N atom, and wherein Cy₁ is optionally substituted by one or more R¹⁰.

In another embodiment the invention relates to the compound of formula **I** as defined above, wherein:
R¹ represents H, -C₁₋₄ alkyl or benzyl, and preferably R¹ represents methyl (-Me), *tert-*butyl (-*t*-Bu) or benzyl (-Bn);
R² to R⁶ represents H,
R⁷ represents -OR⁸;
R⁸ represents C₁₋₄ alkyl, wherein C₁₋₄ alkyl is optionally substituted by one or more R⁹; R⁹ represents Cy₁; and
Cy₁ represents a saturated 5- member ring, which contains 1 or 2 N heteroatoms; wherein Cy₁ is attached to rest of the molecule through any N atom, and wherein Cy₁ is optionally substituted by one or more R¹⁰.

In another embodiment the invention relates to the compound of formula **I** as defined above, wherein:
R¹ represents H, -C₁₋₄ alkyl or benzyl, and preferably R¹ represents methyl (-Me), *tert-*butyl (-*t*-Bu) or benzyl (-Bn);
R² to R⁶ represents H,
R⁷ represents -OR⁸;
R⁸ represents C₁₋₄ alkyl, wherein C₁₋₄ alkyl is optionally substituted by one or more R⁹; R⁹ represents Cy₁; and
Cy₁ represents a saturated 5- member ring, which contains 1 or 2 N heteroatoms; wherein Cy₁ is attached to rest of the molecule through any N atom, and wherein Cy₁ is substituted by one R¹⁰.

In another embodiment the invention relates to the compound of formula **I** as defined above, wherein:
R¹ represents H, -C₁₋₄ alkyl or benzyl, and preferably R¹ represents methyl (-Me), *tert-*butyl (-*t*-Bu) or benzyl (-Bn);
R² to R⁶ represents H,
R⁷ represents -OR⁸;
R⁸ represents C₁₋₄ alkyl, wherein C₁₋₄ alkyl is optionally substituted by one or more R⁹; R⁹ represents Cy₁;
Cy₁ represents a saturated 5- member ring, which contains 1 or 2 N heteroatoms; wherein Cy₁ is attached to rest of the molecule through any N atom, and wherein Cy₁ is substituted by one R¹⁰; and
R¹⁰ represents -C₁₋₄ alkyl optionally substituted by one or more R¹¹, and preferably R¹⁰ represent methyl substituted by one R¹¹.

In another embodiment the invention relates to the compound of formula **I** as defined above, wherein:
R¹ represents H, -C₁₋₄ alkyl or benzyl, and preferably R¹ represents methyl (-Me), *tert-*butyl (-*t*-Bu) or benzyl (-Bn);
R² to R⁶ represents H; and
R⁷ represents H.

In another embodiment the invention relates to the compound of formula **I** as defined above, selected from:
(Z)-*N*-*tert*-Butyl-1-(8-(3-(piperidin-1-yl)propoxy)quinolin-2-yl)methanimine oxide (**3**);
(Z)-*N*-Benzyl-1-(8-(3-(piperidin-1-yl)propoxy)quinolin-2-yl)methanimine oxide (**4**);
(Z)-*N*-*tert*-Butyl-1-(8-(3-(4-(prop-2-yn-1-yl)piperazin-1-yl)propoxy)quinolin-2-yl)methanimine oxide (**7**);
(Z)-*N*-Benzyl-1-(8-(3-(4-(prop-2-yn-1-yl)piperazin-1-yl)propoxy)quinolin-2-yl)methanimine oxide (**8**);
(*Z*)-1-(8-Hydroxyquinolin-2-yl)-*N*-methylmethanimine oxide (**9**);
(*Z*)-*N*-*tert*-butyl-1-(8-hydroxyquinolin-2-yl)methanimine oxide (**10**); and
(Z)-N-Benzyl-1-(8-hydroxyquinolin-2-yl)methanimine oxide (**QN11**).

Some compounds of formula **I** can have chiral centers that can give rise to various stereoisomers. The present invention relates to each of these stereoisomers and also mixtures thereof.

Another aspect of the invention relates to a pharmaceutical composition which comprises a compound of formula **I** as defined above and one or more pharmaceutically acceptable excipients.

As indicated above, the pharmaceutical composition comprises the compound of formula **I** as defined above and one or more pharmaceutically acceptable excipients. The excipients must be "acceptable" in the sense of being compatible with the other ingredients of the composition and not deleterious to the recipients thereof.

Another aspect of the invention relates to a compound of formula **I** as defined above or to a pharmaceutical composition thereof, for use as a medicament.

Another aspect of the invention relates to a compound of formula **I** as defined above or to a pharmaceutical composition thereof, for use for the treatment and/or prevention of a vascular diseases, and preferably wherein the vascular disease is cerebral ischaemia or stroke.

Another aspect of the invention relates to the use of the compound of formula **I** as defined above or to the use of the pharmaceutical composition as defined above, for the manufacture of a medicament.

Another aspect of the invention relates to a method of treating and/or preventing a disease in a subject in need thereof, especially an animal or a human being, which comprises administering to said subject the compound of formula **I** as defined above or the pharmaceutical composition thereof as defined above.

The composition of the present invention can be administered in the form of any pharmaceutical formulation, the nature of which, as it is well known, will depend upon the nature of the active compound and its route of administration. Any route of administration may be used, for example oral, parenteral, nasal, ocular, rectal and topical administration.

The dosage and frequency of doses will depend upon the nature and severity of the disease to be treated, the age, the general condition and body weight of the patient, as well as the composition administered and the route of administration, among other factors.

The compounds of formula **I** as defined above or a pharmaceutical composition thereof, can be administered separately or in combination, particularly as adjuvant therapy administered simultaneously, alternatively or successively with respect to a first-line therapy suitable for the treatment and/or prevention of a vascular diseases, and preferably wherein the vascular disease is cerebral ischemia or stroke. In this sense, the compounds of formula **I** or a pharmaceutical composition thereof administered simultaneously, alternatively or successively with respect to a thrombolytic agent/s and/or thrombectomy procedures, result in particularly suitable therapy for the treatment of a vascular diseases, particularly cerebral ischaemia or stroke.

Thus, another embodiment relates to the compound of formula **I** or the pharmaceutical composition for the use as defined above, which is used in adjuvant therapy and administered simultaneously, alternatively or successively with respect to a first-line therapy aspect, preferably wherein the cardiovascular diseases is cerebral ischemia or stroke, and wherein said first line therapy comprises the use of a thrombolytic agent, preferably the use of recombinant tissue plasminogen activator (rt-PA), and/or thrombectomy procedures.

In the above definitions, the term C₁₋₄ alkyl, as a group or part of a group, means a straight or branched alkyl chain which contains from 1 to 4 carbon atoms and includes, the groups methyl, ethyl, propyl, isopropyl, butyl, isobutyl, sec-butyl and *tert*-butyl.

The term C₂₋₄ alkynyl, as a group or part of a group, means an alkynyl chain which contains from 2 to 4 carbon and includes ethynyl, 1-propynyl, 2-propinyl, 1-butynyl, 2-butynyl and 3-butynyl.

The term "halogen" refers to fluorine, chlorine, bromine, or iodine.

Some of the compounds of the present invention may exist as several diastereoisomers and/or several optical isomers. Diastereoisomers can be separated by conventional techniques such as chromatography or fractional crystallization. Optical isomers can be resolved by conventional techniques of optical resolution to give optically pure isomers. This resolution can be carried out on any chiral synthetic intermediate or on products of formula **I.** Optically pure isomers can also be individually obtained using enantiospecific synthesis. The present invention covers all individual isomers as well as mixtures thereof (for example racemic mixtures or mixtures of diastereomers), whether obtained by synthesis or by physically mixing them.

Additionally, the present invention relates to a method for identifying and evaluating, in a rapid and optionally robotic manner, compounds having high neuroprotective power and involving a possible effective treatment of stroke.

Accordingly, another aspect of the invention relates to the use of a compound of formula **I** or a pharmaceutical composition thereof as defined above, for identifying and evaluating in a robotic manner other compounds having high neuroprotective power which may be useful as a medicament, preferably for the treatment and/or prevention of stroke.

Quinolin-2-yl nitrones of formula **I** and geometric isomers thereof are used to carry out said drug screening. To verify the neuroprotective activity of said quinolin-2-yl nitrones of formula **I** and to enable selecting those compounds with the highest activity, their neuroprotective power is determined using any *in vitro* or *in vivo* model or assay suited to that end. Said models or assays are known for the person skilled in the art; nevertheless, and merely by way of example, a possible assay for determining the neuroprotective activity of quinolin-2-yl nitrones of formula **I** and their possible usefulness in the treatment of vascular diseases, would be in primary neuronal cultures, cultured from 6 to 8 d, taken from the cerebral cortex of rats, where cell viability is determined (Quevedo, C, Salinas, M, Alcázar, A. Initiation factor 2B activity is regulated by protein phosphatase 1, which is activated by the mitogen-activated protein kinase-dependent pathway in insulin-like growth factor 1-stimulated neuronal cells. J. Biol. Chem. 2003, 278, 16579-16586), and subjected to oxygen-glucose deprivation.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

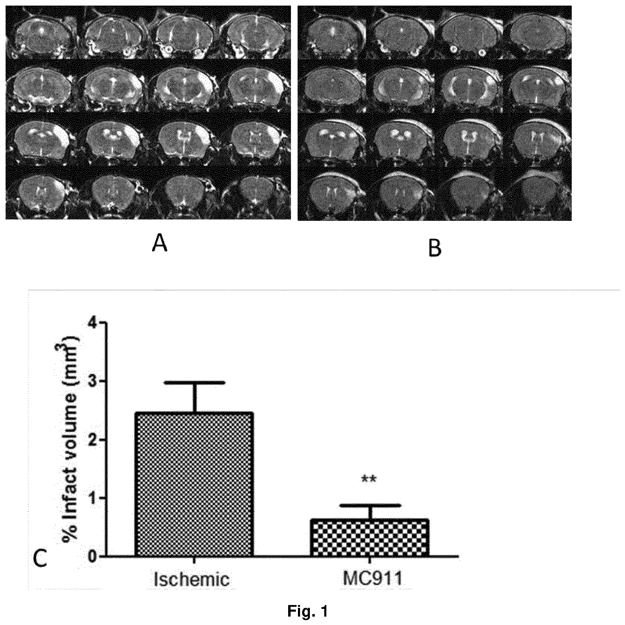

**Fig. 1****.** Shows the MRI study after 48 h of surgery. Note the lesion with T₂ WI hyperintensity restricted to the left side of the cerebral cortex. Treatment with **QN11** reduces the infarct volume after pMCAO in mice. Studies were performed 48 h after pMCAO. T₂WItaken from vehicle treated b group (**A**) and **QN11** c treated group (**B**) randomly chosen ischemic mices are shown. Infarct volumes were quantified from T₂WI using ImageJ free software (**C**). Graphic represents the percentage of the infarct volume with respect to the total volume of the brain. Data are mean ± SEM, n= 7-8. Statistical significance was evaluated by two-way ANOVA followed by post hoc Bonferroni's test to compare paired experimental points (**p < 0.01).

### Examples

### Materials and methods

**General Methods.** Reactions were monitored by TLC using precoated silica gel aluminium plates containing a fluorescent indicator (Merck, 5539). Detection was done by UV (254 nm) followed by charring with sulfuric-acetic acid spray, 1% aqueous potassium permanganate solution or 0.5% phosphomolybdic acid in 95% EtOH. Anhydrous Na₂SO₄ was used to dry organic solutions during workups and the removal of solvents was carried out under vacuum with a rotary evaporator. Flash column chromatography was performed using silica gel 60 (230-400 mesh, Merck). Melting points were determined on a Kofler block and are uncorrected. IR spectra were obtained on a Perkin-Elmer Spectrum One spectrophotometer. ¹H NMR spectra were recorded with a Varian VXR-200S spectrometer, using tetramethylsilane as internal standard and ¹³C NMR spectra were recorded with a Bruker WP-200-SY. All the assignments for protons and carbons were in agreement with 2D COSY, HSQC, HMBC, and 1D NOESY spectra. Values with (*) can be interchanged. The purity of compounds was checked by elemental analyses, conducted on a Carlo Erba EA 1108 apparatus, and confirmed to be ≥95%.

### Example 1: synthesis of nitrones

**General procedure for the synthesis of nitrones.** A solution of the corresponding carbaldehyde (1 mmol), Na₂SO₄ (3 mmol), AcONa (2 mmol) and the appropriate N-alkylhydroxylamine hydrochloride (1.5 mmol) in EtOH (5 mL) was heated at 90 °C for 2-3 h under MWI. After that time, the solvent was evaporated, and the crude mixture was purified on column chromatography using the indicated mixtures of solvents.

Scheme 1 depicted the synthesis of QNs **3** and **4**.

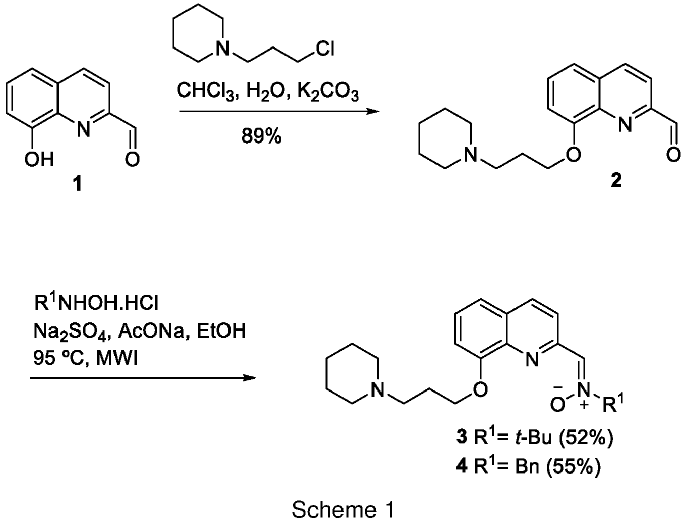

**8-(3-(Piperidin-1-yl)propoxy)quinoline-2-carbaldehyde** (**2**). A solution of commercial 8-hydroxyquinoline-2-carbaldehyde (**1**) (103.9 mg, 0.6 mmol) in CHCl₃ (3.6 mL)/ water (0,6 mL), K₂CO₃ (249 mg, 1.8 mmol) and commercial 1-(3-chloropropyl)piperidine (178.32 mg, 0.9 mmol) were added. The mixture was vigorously stirred and heated at 80 °C for 1 d. After that time, the solvent was evaporated under reduced pressure and the crude mixture was purified on column chromatography (DCM/MeOH 7%) to yield compound **2** as a yellow solid (158.7 mg, 89%): mp 44-6°C; IR (KBr) δ 2932, 1709, 1462, 1323, 1102 cm⁻¹; ¹H NMR (400 MHz, CDCl₃) δ 10.20 (s, 1H), 8.18 (dd, *J* = 8.5, 0.9 Hz, 1H), 7.97 (d, *J* = 8.4 Hz, 1H), 7.52 (t, *J* = 8.0 Hz, 1H), 7.37 (dd, *J* = 8.3, 1.1 Hz, 1H), 7.12 (dd, J = 7.9, 1.2 Hz, 1H), 4.29 (t, *J* = 6.8 Hz, 2H), 2.62 - 2.51 (m, 2H), 2.50 - 2.30 (m, 4H), 2.25 - 2.12 (m, 2H), 1.55 (p, *J* = 5.6 Hz, 4H), 1.39 (q, *J=* 5.7, 4.3 Hz, 2H); ¹³C NMR (101 MHz, CDCl₃) δ 193.9, 155.5, 151.4, 140.1, 137.2, 131.4, 129.8, 119.5, 117.7, 109.9, 67.9, 55.78, 54.6 (2C), 26.33, 25.8 (2C), 24.3. HRMS (ESI_ACN) Calcd. for C₁₈H₂₂N₂O₂: 298.1682. Found: 298.1690.

**(Z)-*N*-*tert*-Butyl-1-(8-(3-(piperidin-1-yl)propoxy)quinolin-2-yl)methanimine oxide (3).** Following the general method for the synthesis of nitrones, a solution of carbaldehyde **2** (79.35 mg, 0.27 mmol), Na₂SO₄ (76.68 mg, 0.54 mmol), AcONa (26.57 mg, 0.324 mmol) and *N*-*tert*-butylhydroxylamine hydrochloride (40.69 mg, 0.324 mmol) in EtOH (5 mL) was heated at 95 °C for 3 h under MWI. Then, the solvent was evaporated and the crude mixture was purified on column chromatography (DCM/MeOH 7%) to yield compound **3** as a yellow solid (52.3 mg, 52%): mp 135-7 °C; IR (KBr) δ 3493, 2942, 1615, 1261, 1096 cm⁻¹; ¹H NMR (400 MHz, CDCl₃) δ 9.27 (d, *J* = 8.8 Hz, 1H), 8.36 - 8.13 (m, 1H), 7.53 - 7.41 (m, 2H), 7.16 -7.04 (m, 1H), 4.37 (t, *J* = 5.7 Hz, 2H), 3.61 (d, *J* = 11.9 Hz, 2H), 3.43 - 3.28 (m, 2H), 2.68 (dd, *J* = 15.8, 6.7 Hz, 4H), 2.32 (d, *J* = 13.8 Hz, 2H), 2.00 - 1.79 (m, 4H), 1.67 (s, 9H); ¹³C NMR (101 MHz, CDCl₃) δ 137.5, 132.1, 129.6, 127.6, 127.5, 121.9, 120.4, 119.1, 110.2, 110.0, 72.5. 66.7, 55.4, 53.5 (2C), 28.4 (3C), 23.9, 22.7 (2C), 22.2. HRMS (ESI_ACN) Calcd. for C₂₂H₃₁N₃O₂: 369.24163. Found: 369.24136.

**(Z)-*N*-Benzyl-1-(8-(3-(piperidin-1-yl)propoxy)quinolin-2-yl)methanimine oxide (4).** Following the general method for the synthesis of nitrones, a solution of carbaldehyde **2** (79.35 mg, 0.27 mmol), Na₂SO₄ (76.68 mg, 0.54 mmol), AcONa (26.57 mg, 0.324 mmol) and *N*-benzylhydroxylamine hydrochloride (51.71 mg, 0.324 mmol) in EtOH (5 mL) was heated at 90 °C for 2 h under MWI. After that time, the solvent was evaporated and the crude mixture was purified on column chromatography (DCM/MeOH 7%) to yield compound **4** as a mixture of Z and E isomers in a 3.5:1 ratio, that were unable to separate, as a yellow solid (59.7 mg, 55%): mp 108-10 °C; IR (KBr) δ 3420, 2935, 1600, 1455, 1105 cm⁻¹; ¹H NMR (400 MHz, CDCl₃) **[(minor isomer *E***) 8.17 (d, *J* = 8.8 Hz, 1H, H4), 7.79 (s, 1H, CH=N), 7.74 (dd, *J* = 8.4, 1.1 Hz, 1H, H3), 7.55 (dd, *J*= 7.0, 2.5 Hz, 1H, H5), 7.09 (br d, *J*= 1.5 Hz, 1H, H7), 5.43 (s, 1H, C*H*₂C₆H₅), (**major isomer *Z***) 9.15 (d, *J* = 8.8 Hz, 1H, H4), 8.17 (d, *J* = 8.8 Hz, 1H, H3), 8.08 (s, 1H, CH=N), 7.07 (br d, *J*= 7.5 Hz, 1H, H7), 5.15 (s, 1H, C*H*₂C₆H₅)], 8.28 - 8.26 (m, 1H, C₆H₅), 7.54 (br d, *J=* Hz, 1H, H5), 7.44 - 7.38 (m, 6H, H6, C₆H₅), 7.45 (t, *J=* Hz, 1H, H6), 7.52 - 7.37 (m, 4H, C₆H₅), 4.31 (t, *J=* Hz., 2H, OCH₂), 3.15 - 2.65 (m, 6H), 2.43-2.41 (m, 2H), 1.98-1.70 (m, 4 H), 1.65-1.51 (m, 2H). HRMS (ESI_ACN) Calcd. for C₂₅H₂₉N₃O₂: 403.22598. Found: 403.22528.

Scheme 2 depicted the synthesis of QNs **7** and **8**.

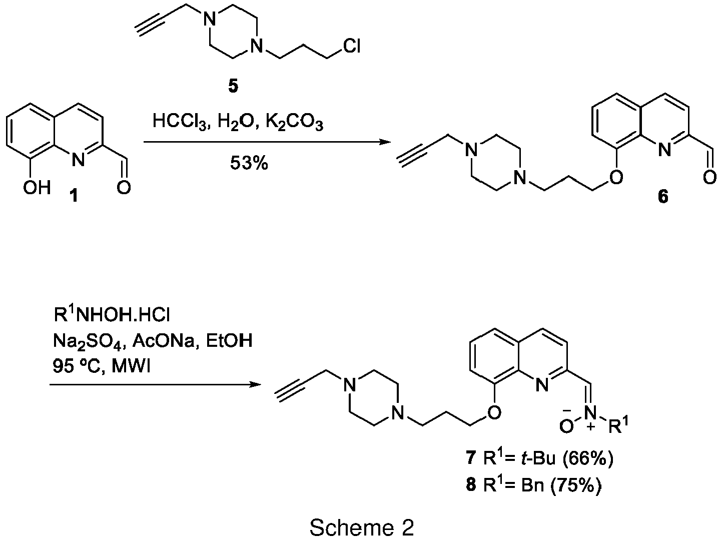

**1-(3-Chloropropyl)-4-(prop-2-yn-1-yl)piperazine** (**5**). To solution of commercial 1-(3-chloropropyl)piperazine dihydrochloride (702 mg, 3 mmol, 1 equiv), TEA (0,84 mL, 6 mmol, 2 equiv) in dry CH₂Cl₂ (5 mL), cooled at 0 °C, propargyl bromide (0,81 mL, 9 mmol, 3 equiv) was added over 30 min under Ar. The mixture was stirred at rt for 24 h and then treated with a saturated sodium bicarbonate solution (10 mL). The organic layer was separated and washed with saturated brine, and dried over anhydrous sodium sulfate. The solvent was removed under reduced pressure and the residue purified by column chromatography through silica gel (CH₂Cl₂/MeOH 1%-2%), to yield **5** (312 mg, 52%) as a colorless oil: ¹H NMR (400 MHz, CDCl₃) δ 3.53 (t, *J* = 6.6 Hz, 2H), 3.23 (d, *J* = 2.5 Hz, 2H), 2.54 - 2.39 (m, 10H), 2.18 (t, *J* = 2.5 Hz, 1H), 1.88 (p, *J* = 6.7 Hz, 2H); ¹³C NMR (101 MHz, CDCl₃) δ 78.8, 73.2, 55.4, 53.1 (2C), 51.9 (2C), 46.8, 43.2, 29.9.

**8-(3-(4-(Prop-2-yn-1-yl)piperazin-1-yl)propoxy)quinoline-2-carbaldehyde (6).** A solution of commercial 8-hydroxyquinoline-2-carbaldehyde (**1**) (91.69 mg, 0.53 mmol) in CHCl₃ (5 mL) was added K₂CO₃ (219.42 mg, 1.59 mmol) and 1-(3-chloropropyl)-4-(prop-2-yn-1-yl)piperazine (**5**) (138 mg, 0.69 mmol); then, water (1 mL) was added. The mixture was vigorously stirred and heated at 80 °C for 2 d. After that time, the solvent was evaporated under reduced pressure and the crude mixture was purified on column chromatography (DCM/MeOH 4%) to yield compound **6** as a yellow solid (95.5 mg, 53%): mp 99-101 °C; IR (KBr) δ 3128, 2829, 1715, 1462, 1320, 1094 cm⁻¹; ¹H NMR (400 MHz, CDCl₃) δ 10.20 (s, 1H), 8.20 (d, *J* = 8.4 Hz, 1H), 7.98 (d, *J* = 8.5 Hz, 1H), 7.53 (t, *J* = 8.5 Hz, 1H), 7.39 (d, *J* = 8.5 Hz, 1H), 7.13 (d, *J* = 8.4 Hz, 1H), 4.31 (t, *J=* 6.5 Hz, 2H), 3.24 (s, 2H), 2.76 - 2.36 (m, 10H), 2.26 - 2.13 (m, 3H); ¹³C NMR (101 MHz, CDCl₃) δ 193.8, 155.5, 151.4, 140.1, 137.2, 131.4, 129.8, 119.6, 117.8, 110.0, 78.8, 73.2, 67.7, 55.0, 53.0 (2C), 51.8 (2C), 46.8, 26.3. HRMS (ESI_ACN) Calcd. for C₂₀H₂₃N₃O₂: 337.1790. Found: 337.1790.

**(Z)-*N*-*tert*-Butyl-1-(8-(3-(4-(prop-2-yn-1-yl)piperazin-1-yl)propoxy)quinolin-2-yl)methanimine oxide (7).** Following the general method for the synthesis of nitrones, the reaction of carbaldehyde **6** (69 mg, 0.20 mmol) with Na₂SO₄ (57 mg, 0.4 mmol), AcONa (26 mg, 0.32 mmol) and *N*-*tert*-butylhydroxylamine hydrochloride (38 mg, 0.3 mmol) in EtOH (5 mL) after 5 min, and column chromatography (DCM/MeOH 3%) yielded compound **7** as a yellow gum (55.7 mg, 66%): IR (KBr) δ 3428, 2819, 1451, 13206, 1155, 1104 cm⁻¹; ¹H NMR (400 Hz, CDCl₃) δ 9.26 (d, *J* = 8.8 Hz, 1H), 8.19 (d, *J* = 8.8 Hz, 1H), 8.13 (s, 1H), 7.44-7.38 (m, 2H,), 7.08-7.06 (m, 1H), 4.29 (t, *J* = 6.9 Hz, 2H), 3.30-3.28 (m, 2H), 2.63 (s, 10H), 2.32 - 2.17 (m, 3H), 1.65 (s, 9H); ¹³C NMR (126 MHz, CDCl₃) δ 154.6, 149.8, 140.2, 136.6, 132.6, 129.5, 127.3, 121.7, 119.5, 109.0, 78.7, 73.4, 72.0, 67.7, 55.0, 53.0 (2C), 51.8 (2C), 46.8, 28.3 (3C), 26.3. HRMS (ESI_ACN) Calcd for C₂₄H₃₂N₄O₂: 408,2525. Found: 408,2525.

**(Z)-*N*-*Benzyl*-1-(8-(3-(4-(prop-2-yn-1-yl)piperazin-1-yl)propoxy)quinolin-2-yl)methanimine oxide (8).** Following the general method for the synthesis of nitrones, a solution of carbaldehyde **6** (95.5 mg, 0.28 mmol), treated with Na₂SO₄ (79.52 mg, 0.56 mmol), NaHCO₃ (35.28 mg, 0.42 mmol) and *N*-benzylhydroxylamine hydrochloride (67.03 mg, 0.42 mmol), in THF (5 mL), reacted instantly. After that time, the solvent was evaporated and the crude mixture was purified on column chromatography (DCM/MeOH 3%) to yield compound **8** as a mixture of E and Z isomers in a 1.5:1 ratio, that were unable to separate, as a yellow gum (92.5 mg, 75%): IR(KBr) δ 3429, 2817, 1457, 1320, 1151, 1104 cm⁻¹; ¹H NMR (500 MHz, CDCl₃) δ **[(major isomer *E***) 8.17 (d, *J* = 8.5 Hz, 1H, H4), 7.81 (d, *J* = 8.5 Hz, 1H, H3), 7.75 (s, 1H, CH=N), 7.55 (dd, *J*= 7.8, 1.7 Hz, 1H, H5), 7.12 (dd, *J*= 7.7, 1.2 Hz, 1H, H7), 5.44 (s, 1H, C*H*₂C₆H₅), 3.30 (d, *J* = 2.4 Hz, 2H, CH₂C≡CH), (**minor isomer *Z***) 9.18 (d, *J* = 8.7 Hz, 1H, H4), 8.19 (d, *J* = 8.7 Hz, 1H, H3), 8.06 (s, 1H, CH=N), 7.10 (dd, *J*= 7.7, 1.3 Hz, 1H, H7), 5.14 (s, 1H, C*H*₂C₆H₅), 3.31 (d, *J* = 2.4 Hz, 2H, C*H*₂C≡CH)], 8.26 - 8.24 (m, 1H, C₆H₅), 7.45 (t, *J*= Hz, 1H, H6), 7.52 - 7.37 [m, 4H, H5 (minor)], C₆H₅), 4.31 (t, *J*= Hz, 2H, OCH₂), 2.75 - 2.50 (m, 10H), 2.25-2.24 (m, 1H, CH₂C≡C*H*), 2.24-2.16 (h, *J=* Hz, 2H, NCH₂C*H*₂CH₂O). HRMS (ESI_ACN) Calcd for C₂₇H₃₀ClN₄O₂: 442.2369. Found: 442.2369.

Scheme 3 depicted the synthesis of of compounds **9**, **10** and **QN11**.

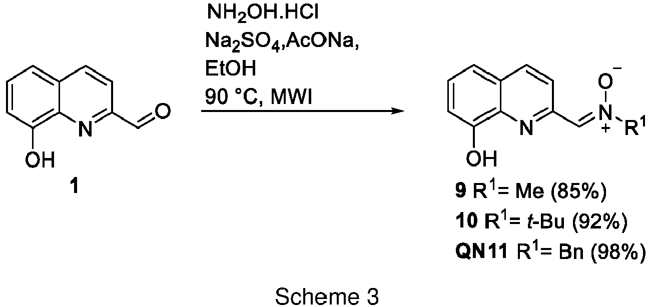

**(*Z*)-1-(8-Hydroxyquinolin-2-yl)-*N*-methylmethanimine oxide** (**9**). Following the general method for the synthesis of nitrones, the reaction of 8-hydroxyquinoline-2-carbaldehyde (**1**) (173 mg, 1 mmol, 1 equiv) with Na₂SO₄ (426 mg, 3 mmol, 3 equiv), AcONa (160 mg, 2 mmol, 1.6 equiv) and *N*-methylhydroxylamine hydrochloride (239 mg, 1.5 mmol, 1.5 equiv) in EtOH (7 mL) at 90 °C for 10 min, after column chromatography (hexane/AcOEt 9/1) yielded compound **9** as a pale yellow solid (171 mg, 85%): 149-151 ºC; ¹H NMR (300 MHz, CDCl₃) δ 9.16 (d, *J* = 8.8 Hz, 1H), 8.25 (d, *J* = 8.8 Hz, 1H), 8.03 (br s, 1H), 7.86 (s, 1H), 7.51-7.46 (m, 1H), 7.36 (dd, *J* = 7.6, 1.3 Hz, 1H), 7.18 (dd, *J* = 7.6, 1.3 Hz, 1H), 4.01 (s, 3H); ¹³C NMR (100 MHz, CDCl₃) δ 152.1, 147.7, 138.1, 136.8, 136.4, 128.7, 128.6, 121.4, 117.8, 110.1, 55.1. HRMS (ESI_ACN) Calcd. for C₁₁H₁₀N₂O₂: 202,0742. Found 202,0742.

**(*Z*)-*N*-*tert*-butyl-1-(8-hydroxyquinolin-2-yl)methanimine oxide (10).** Following the general method for the synthesis of nitrones, the reaction of 8-hydroxyquinoline-2-carbaldehyde (**1**) (173 mg, 1 mmol, 1 equiv) with Na₂SO₄ (426 mg, 3 mmol, 3 equiv), AcONa (160 mg, 2 mmol, 1.6 equiv) and *N*- *tert*-butylhydroxylamine hydrochloride (188 mg, 1.5 mmol, 1.5 equiv) in EtOH (7 mL) at 90 °C for 5 min, after column chromatography (hexane/AcOEt 9/1), yielded compound **10** as a pale yellow solid (159 mg, 92%): 103-4 ºC; ¹H NMR (400 MHz, CDCl₃) δ 9.16 (d, *J=* 8.8 Hz, 1H), 8.16 (d, *J* = 8.8 Hz, 1H), 8.04 (br s , 1H), 7.97 (s, 1H), 7.41-7.39 (m, 1H), 7.27 (dd, *J* = 7.6, 1.2 Hz, 1H), 7.09 (dd, *J* = 7.6, 1.2 Hz, 1H), 1.62 (s, 9H); ¹³C NMR (100 MHz, CDCl₃) δ 152.0, 148.5, 138.1, 136.7, 131.5, 128.5, 128.4, 121.7, 117.8, 110.0, 72.1, 28.4 (3C). HRMS (ESI_ACN) Calcd. for C₁₄H₁₆N₂O₂: 244,1212. Found 244,1212.

**(*Z*)-*N*-Benzyl-1-(8-hydroxyquinolin-2-yl)methanimine oxide (QN11).** Following the general method for the synthesis of nitrones, the reaction of 8-hydroxyquinoline-2-carbaldehyde (**1**) (173 mg, 1 mmol, 1 equiv), Na₂SO₄ (426 mg, 3 mmol, 3 equiv.), AcONa (160 mg, 2 mmol, 1.6 equiv.) and *N*-benzylhydroxylamine hydrochloride (239 mg, 1.5 mmol, 1.5 equiv.) in EtOH (7 mL) was heated at 90°C during 10 min under mw irradiation. After that time, the solvent was evaporated and the crude mixture was purified on column chromatography (Hexanes/AcOEt 9/1) to yield compound **QN11** as a pale yellow solid (272 mg, 98%): 110-1 ºC; ¹H NMR (400 MHz, CDCl₃) δ 9.15 (d, *J* = 8.8 Hz, 1H), 8.22 (d, *J* = 8.8 Hz, 1H), 8.00 (br s, 1H), 7.88 (s, 1H), 7.55-7.51(m, 2H), 7.44-7.37 (m, 4H), 7.26 (dd, *J* = 7.6, 1.2 Hz, 1H), 7.08 (dd, *J* = 7.6, 1.2 Hz, 1H), 5.09 (s, 2H); ¹³C NMR (100 MHz, CDCl₃) δ 152.1, 147.7, 138.1, 136.8, 135.3, 132.7, 129.5 (2C), 129.3, 129.2 (2C), 128.7, 128.6, 121.6, 117.8, 110.1, 72.1. HRMS (ESI_ACN) Calcd. for C₁₇H₁₄N₂O₂: 278,1055. Found 278,1055.

### Example 2: pMCAO stroke model

### Methods and results

To study of the effects of **QN11** on stroke recovery, administration of the vehicle and **QN11** molecule was performed intraperitoneally, to 8-week-old male C57BL/6J mice (Harlan) weighing 25-30 g. All procedures with animals were carried out under a protocol approved by the Ethical Committee of the Spanish National Research Council (CSIC), and recommendations of the European Council. A special effort was made to keep to a minimum necessary the number of animals to achieve adequate significance. For surgery, anaesthesia induction was carried out with 3% isoflurane (in 70% N₂O, 30% O₂), followed by 2% isoflurane for maintenance during stroke procedure. Rectal temperature was maintained at 36.5 ºC with the use of a heating pad. The common branch of the middle cerebral artery was, after craniotomy, exposed and occluded permanently by suture ligation. To ensure a complete artery occlusion during surgery, cortical blood flow was monitored by non-invasive laser Doppler flowmetry, as a quality control, with the aid of a Perimed equipment (PeriFlux System 5000 Stroke Model Monitor, Perimed, Järfälla, Sweden). The study was exclusively performed in animals that showed post-ligature a drop of blood flow of at least 65%. Animals subjected to surgery for longer than 15 min were excluded of the study. Physiological parameters were maintained. Experiments were performed in each of the following groups: (a) sham operated (n= 6 animals); (b) pMCAO vehicle control group (saline buffer containing 29% dimethyl sulfoxide, DMSO) (n= 7), and (c) pMCAO **QN11** treated group (85 mg/kg **QN11** dissolved in vehicle) (n= 8). Drug administration was 15 min after arterial ligature.

MRI is the most commonly used diagnostic tool for stroke. Diffusion-weighted images (WI) were acquired in T₂ (T₂WI) as a tool to obtain precise images of the vascular edema, with a resolution limit of 0.8-1 mm³. This information is needed to delineate lesion boundaries for assessment of intracranial edema location and growth pattern evaluation. In this study, determination of infarct size in vehicle and **QN11** treated mice was performed in sequential MRI coronal 0.8-1 mm-thick brain slices (Figures 1A, 1B). MRI studies were performed at BiolmaC (ICTS Biolmagen Complutense), node of the ICTS ReDIB (https://www.redib.net/) using one tesla (1 T) benchtop MRI scanner ICON-1T; Bruker BioSpin (GmbH, Ettlingen, Germany). The system consists of a 1 T permanent magnet (without extra cooling required for the magnet) with a gradient coil that provides a gradient strength of 450 mT/m. The animal monitoring systems and the solenoid mouse head RF are integrated into the bed and they allow the animals to be handled on with accurate positioning of the coil and full control of anaesthesia and body temperature. MRI experiment consisted of three dimensional T₂WI used to evaluate the vascular edema. Three-dimensional T₂WI were acquired using a rapid acquisition with relaxation enhancement (RARE) technique, with a repetition time (TR) = 2500 s, echo train length = 12, interecho interval = 18 ms (resulting in an effective echo time (TE) = 90 ms), number of average = 1, field of view (FOV) = 18 x 18 x 14 mm. The acquired matrix size was 120x 120 x 28 (resolution 0.150 x 0.150 x 0.500 mm) and total acquisition time was ~ 12 min. All MRI data was analysed using ImageJ software.

The experiments compared infarct volume outcome between group's b and c (Figures 1A, 1B, respectively). Sham operated control group (a) showed with certainty that stroke was not due to the surgical pre-occlusive procedure. Infarct volumes, shown in mm³ were obtained integrating infarcted areas by counting pixels contained within the regions of interest. Each side of the coronal sections was sampled. Compared with vehicle-treated animals (b), a significant reduction in the percentage of brain infarct volume (74,47%) was appreciated in **QN11** treated group (c) (Figure 1C). With regard to the whole brain volume, the infarct volume in group b represented 2.453 %, whereas in group a it dropped to 0.6262%. Statistical significance was evaluated by two-way ANOVA followed by post hoc Bonferroni's test Student to determine the statistical significance of differences of infarct values between vehicle (b) and the **QN11** treated (c) mice. P value < 0.05 was considered significant.

These results show evidence which suggest that quinolylnitrone **QN11** is a therapeutic target for stroke, showing easy and efficient penetration of the compound through the BBB (Brain Blood Barrier).

## Claims

1. A compound of formula **I**: 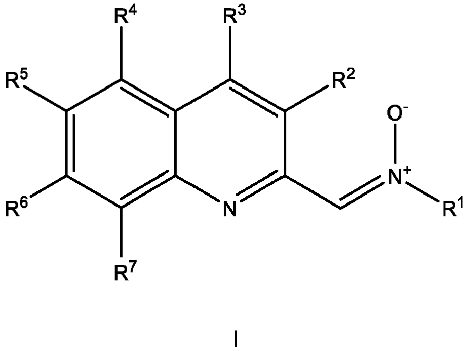 wherein:
R¹ independently represents C₁₋₄ alkyl, phenyl or benzyl (Bn);
each R² to R⁷ independently represents H, C₁₋₄ alkyl, phenyl, halogen, -OR⁸ or -NHR⁸;
each R⁸ independently represents H or C₁₋₄ alkyl, wherein C₁₋₄ alkyl is optionally substituted by one or more R⁹.
each R⁹ independently represents halogen, -OH, -OC₁₋₄ alkyl, NH₂, -NH(C₁₋₄ alkyl) or Cy₁; and
Cy₁ represents a 5-to-8-member ring, saturated, partially unsaturated or
aromatic, which optionally contains from 1 to 3 heteroatoms selected from N, O, Se and/or S; wherein Cy₁ may be attached to rest of the molecule through any available C or N atom, and wherein Cy₁ is optionally substituted by one or more R¹⁰;
each R¹⁰ independently represent -C₁₋₄ alkyl optionally substituted by one or more R¹¹; and
each R¹¹ independently represents -C₂₋₄ alkynyl.

2. The compound of formula **I** according to claim 1, wherein R¹ represents H, -C₁₋₄ alkyl or benzyl, and preferably R¹ represents methyl (-Me), *tert*-butyl (-*t*-Bu) or benzyl (-Bn).

3. The compound of formula **I** according to any of claims 1 or 2, wherein each R² to R⁶ independently represents H.

4. The compound of formula **I** according to any of claims 1 to 3, wherein R⁷ represents H or -OR⁸.

5. The compound of formula **I** according to any of claims 1 to 4, wherein R⁸ represents C₁₋₄ alkyl optionally substituted by one or more R⁹.

6. The compound of formula **I** according to any of claims 1 to 5, wherein R⁹ represents Cy₁.

7. The compound of formula **I** according to any of claims 1 to 6, wherein Cy₁ represents a saturated 5- member ring, which contains 1 or 2 N heteroatoms; wherein Cy₁ may be attached to rest of the molecule through any available C or N atom, and wherein Cy₁ is optionally substituted by one or more R¹⁰.

8. The compound of formula **I** according to any of claims 1 to 7, wherein R¹⁰ represents -C₁₋₄ alkyl optionally substituted by one or more R¹¹.

9. The compound of formula **I** according to claim 1, selected from:
(Z)-*N*-*tert*-Butyl-1-(8-(3-(piperidin-1-yl)propoxy)quinolin-2-yl)methanimine oxide (**3**);
(Z)-*N*-Benzyl-1-(8-(3-(piperidin-1-yl)propoxy)quinolin-2-yl)methanimine oxide (**4**);
(Z)-*N*-*tert*-Butyl-1-(8-(3-(4-(prop-2-yn-1-yl)piperazin-1-yl)propoxy)quinolin-2-yl)methanimine oxide (**7**);
(Z)-*N*-Benzyl-1-(8-(3-(4-(prop-2-yn-1-yl)piperazin-1-yl)propoxy)quinolin-2-yl)methanimine oxide (**8**);
(*Z*)-1-(8-Hydroxyquinolin-2-yl)-*N*-methylmethanimine oxide (**9**);
(*Z*)-*N*-*tert*-butyl-1-(8-hydroxyquinolin-2-yl)methanimine oxide (**10**); and
(*Z*)-*N*-Benzyl-1-(8-hydroxyquinolin-2-yl)methanimine oxide (**QN11**).

10. A pharmaceutical composition which comprises a compound of formula **I** according to any of claims 1 to 9 and one or more pharmaceutically acceptable excipients.

11. A compound of formula **I** according to any of claims 1 to 9 or a pharmaceutical composition according to claim 10, for use as a medicament.

12. A compound of formula **I** according to any of claims 1 to 9 or a pharmaceutical composition according to claim 10, for use for the treatment and/or prevention of a vascular diseases.

13. The compound of formula **I** or the pharmaceutical composition for the use according to claim 12, wherein the vascular disease is cerebral ischaemia or stroke.

14. The compound of formula **I** or the pharmaceutical composition for the use according to any of claims 12 or 13, which is used in adjuvant therapy and administered simultaneously, alternatively or successively with respect to a first-line therapy.

15. The compound of formula **I** or the pharmaceutical composition for the use according to claim 14, wherein said first line therapy comprises the use of a thrombolytic agent, preferably the use of recombinant tissue plasminogen activator (rt-PA), and/or thrombectomy procedures.
